# EUROPÄISCHE PATENTSCHRIFT

(11) **EP 2 779 963 B1**
(45) Veröffentlichungstag und Bekanntmachung des Hinweises auf die Patenterteilung: **20.02.2019**
(21) Anmeldenummer: 12790794.7
(22) Anmeldetag: 16.11.2012
(51) Int. Cl.: A61F 5/01, A61F 2/78

(54) **ORTHOPÄDIETECHNISCHE MANSCHETTE UND VERFAHREN ZU DEREN HERSTELLUNG**
ORTHOPAEDIC SLEEVE AND METHOD FOR PRODUCING THE SAME
MANCHETTE CONÇUE SELON UNE TECHNIQUE ORTHOPÉDIQUE ET SON PROCÉDÉ DE PRODUCTION

(30) Priorität: 16.11.2011 DE 102011118617
(43) Veröffentlichungstag der Anmeldung: 24.09.2014
(73) Patentinhaber: Ottobock SE & Co. KGaA, 37115 Duderstadt (DE)
(72) Erfinder: REINHARDT, Holger, 47906 Kempen (DE); VOLLBRECHT, Matthias, 37412 Herzberg (DE)
(74) Vertreter: Stornebel, Kai
(86) Internationale Anmeldenummer: PCT/EP2012/004774
(87) Internationale Veröffentlichungsnummer: WO 2013/072064

(56) Entgegenhaltungen:
- WO-A1-01/60289
- US-A- 5 713 837
- US-A1- 2005 240 283
- US-A1- 2008 294 082

## Beschreibung

Die Erfindung betrifft eine orthopädietechnische Manschette zur Anlage an einem Körper aus einem flexiblen Grundkörper mit zumindest einem daran angeordneten Stabilisierungselement aus Kunststoff sowie ein Verfahren zur Herstellung einer solchen Manschette. Die Manschette ist insbesondere als ein Liner als Interface zwischen einem Stumpf und einem Prothesenschaft ausgebildet, kann aber auch als eine Bandage ausgebildet sein, die zur Abpolsterung und/oder Stützung an dem Körper angelegt wird.

Bandagen, insbesondere Gelenkbandagen, werden zur Unterstützung oder zum Schutz von Muskulatur und Bändern angelegt. Dazu wird die Bandage über das zu schützende Gelenk gezogen oder darum gewickelt und gegebenenfalls fixiert. Bandagen lassen, im Gegensatz zu Immobilisationsorthesen, eine Bewegung der durch das Gelenk verbundenen Gliedmaßen relativ zueinander in einem gewissen Umfang in zumindest einem Freiheitsgrad zu. Dazu weisen die Bandagen einen Grundkörper auf, der aus einem flexiblen Material besteht, beispielsweise einem Schaumstoff oder einem Textil. Zur Unterstützung der Stabilisationswirkung sind an dem Grundkörper Stabilisierungselemente angeordnet, die eine gegenüber dem Grundkörper erhöhte Festigkeit und Steifigkeit aufweisen, so dass neben einem Schutz gegen mechanische Einwirkungen eine erhöhte Bewegungseinschränkung bewirkt wird.

Weiterhin sind sogenannte Liner bekannt, die an einem Stumpf einer Gliedmaße angelegt werden, um eine Schnittstelle zu einer Prothese herzustellen. Die Liner können als Kunststoff- oder Silikonliner ausgebildet sein und auf der Innenseite luftdicht oder haftend beschichtet oder ausgebildet sind. Über Unterdruck hält der Liner einen Prothesenschaft an dem Stumpf, wobei der Unterdruck zwischen dem Liner und dem Prothesenschaft anliegen kann. Alternativ weist der Liner auf seiner Außenseite Verriegelungselemente auf, über die der Prothesenschaft an dem Liner gehalten wird. Der Liner haftet an dem Stumpf aufgrund der Beschaffenheit seiner inneren Oberfläche.

Die US 2008/0039757 A1 beschreibt eine flexible Bandage mit einem Rahmen mit einer Oberfläche zum Anlegen an einen Körperbereich, wobei der Rahmen eine Vielzahl permanenter Öffnungen aufweist. An dem Rahmen kann ein Gewebe oder Schaumstoff angebracht sein, beispielsweise durch Verschweißen, Kleben oder Nähen.

Die EP 876 130 B1 beschreibt eine orthopädische Stütze mit einer Halterung aus einem einteiligen Spritzgussteil mit Bereichen unterschiedlicher Dicken, um sich dem Umriss eines ausgewählten Teils eines Körpergliedes anzupassen. Eine Lage elastisches Material ist innerhalb der Halterung angebracht, um die Halterung zu polstern, wenn sie am ausgewählten Teil des Körpergliedes angreift. Die Halterung kann angeklebt oder auf andere Art und Weise mit der Polsterung verbunden sein.

Die US 6,024,712 beschreibt eine Sprunggelenkorthese mit einem inneren Textilteil, das sich zumindest teilweise um das Sprunggelenk erstreckt. Eine Außenstütze ist in dem Textil über Spritzgießen eingeformt und stellt eine zusätzliche Abstützung des Gelenks gegen unbeabsichtigte Bewegungen dar.

Die CA 2,398,059 A1 beschreibt eine orthopädische Stütze mit einem flexiblen Innenteil und einer Außenstütze, die direkt auf das flexible innere Teil aufgeformt ist.

Die US 2005/0240283 A1 betrifft einen Liner mit Anschlussmitteln zum Verbinden externer Vorrichtungen an dem Liner. Solche Anschlussmittel beinhalten Pins, Kabel, Bänder, Klettverschlüsse, Schnallen, Knöpfe usw. und dienen üblicherweise zur Befestigung und zur Abstützung einer prothetischen Einrichtung.

Die US 5,713,837 A betrifft eine einstückige Orthese mit einem flexiblen Grundkörper und mehreren darauf aufgebrachten Stabilisierungselementen, wobei die Manschette Bereiche aufweist, die einen gegenüber dem übrigen Querschnitt verringerten Querschnitt aufweisen. In den Bereichen des verringerten Querschnittes sind die Stabilisierungselemente mit dem Grundkörper stoffschlüssig verbunden.

Die US 2008/0294082 A1 betrifft eine Fußheberorthese mit einem flexiblen Grundkörper und einem daran angeordneten Stabilisierungselement aus Kunststoff. Das Stabilisierungselement ist auf dem Grundkörper aufgeklebt oder stoffschlüssig mit dem Grundkörper verbunden.

Die WO 01/60289 A1 betrifft eine orthopädische Stützeinrichtung mit einem flexiblen Innenteil und einem Exoskelett, das direkt auf das flexible Innenteil aufgeformt ist. Die Stützeinrichtung kann als Handgelenkstütze mit einem Daumenloch ausgebildet sein. Statt des direkten Anformens ist alternativ vorgesehen, dass die Exostruktur auf dem flexiblen Material entfernbar über Befestigungsmittel anordbar ist, beispielsweise über Klettverschlüsse, Druckknöpfe oder dergleichen.

Problematisch ist, dass die direkte Anbindung des Polstermaterials an den Stabilisierungselementen zu Spannungssprüngen führt, was hinsichtlich der Haltbarkeit und des Tragekomforts nachteilig ist.

Aufgabe der vorliegenden Erfindung ist es, eine orthopädietechnische Manschette bereitzustellen, bei der ein fester und haltbarer Verbund zwischen dem Grundkörper und dem Stabilisierungselement erreicht wird.

Erfindungsgemäß wird diese Aufgabe durch eine Manschette mit den Merkmalen des Hauptanspruches und ein Verfahren mit den Merkmalen des nebengeordneten Anspruchs erreicht. Vorteilhafte Ausgestaltungen und Weiterbildungen der Erfindung sind in den Unteransprüchen, der Beschreibung und den Figuren offenbart.

Die orthopädietechnische Manschette, die zur Anlage an einen Körper vorgesehen ist, mit einem flexiblen Grundkörper mit zumindest einem daran angeordneten Stabilisierungselement aus Kunststoff, das auf einem flexiblen Trägermaterial aufgebracht ist, das auf der dem Grundkörper zugewandten Seite mit einem mit dem Grundkörper verschweißbaren oder verklebbaren Kunststoff kaschiert ist, sieht vor, dass das Trägermaterial über den Umfang des Stabilisierungselementes zumindest abschnittsweise hinaussteht und nur in über den Umfang des Stabilisierungselementes hinausstehenden Bereichen des Trägermaterials eine Verklebung oder andere stoffschlüssige Verbindung mit dem Grundkörper ausgeführt ist. Durch die Verbindung in den Bereichen mit einem verringerten Querschnitt ist es möglich, Verbindungsbereiche mit ausreichender Elastizität bereitzustellen, so dass die Gefahr von Rissen oder Ablösungen des Grundkörpers von dem Stabilisierungselement verringert wird. Es wird also zumindest ein Übergang durch eine Querschnittsverringerung bereitgestellt, so dass in den Randbereichen des Stabilisierungselementes eine gegenüber dem übrigen Querschnitt erhöhte Flexibilität vorhanden ist, die die Gefahr eines Ausreißens der Verbindung zu dem Grundkörper oder dergleichen verringert. Die Manschette kann Teil einer Orthese oder Prothese sein und darüber hinaus in ein System orthetischer oder prothetischer Komponenten eingebunden sein. Ebenfalls kann die Manschette eine eigenständige Stütz- und Schutzfunktion ausüben, wenn sie als Bandage getragen wird

Die Erfindung sieht somit vor, dass das Stabilisierungselement auf einem flexiblen Trägermaterial aufgebracht ist. Dieses Aufbringen kann durch Aufspritzen oder Hinterspritzen des Stabilisierungselementes an dem Trägermaterial erfolgen. Das Trägermaterial bildet dann wiederum diejenigen Bereiche aus, die stoffschlüssig mit dem Grundkörper verbunden oder aufgeklebt sind. Durch die Zwischenschaltung des Trägermaterials bei der Befestigung des Stabilisierungselementes oder der Stabilisierungselemente auf dem Grundkörper ist es möglich, die Stabilisierungselemente sehr haltbar an das Trägermaterial zu binden und dieses dann wiederum mit dem Grundkörper zu verbinden, beispielsweise zu verschweißen, aufzukleben oder dergleichen. Mit einem solchen aufgespritzten Stabilisierungselement ist eine hohe Reproduzierbarkeit der Verbindung möglich, ebenfalls ist das flexible Trägermaterial in der Lage, eine wenn auch kleine Verlagerbarkeit des Stabilisierungselementes relativ zu dem Grundkörper zu ermöglichen, wodurch Risse in Übergängen von dem Stabilisierungselement zu dem Trägermaterial und von dem Trägermaterial zu dem Grundkörper vermieden werden können. Auch ist es möglich, gelenkige Verbindungen durch Aufspritzen oder Hinterspritzen an dem Trägermaterial zu realisieren, beispielsweise indem zwei Stabilisierungselemente so einander gegenüberliegend angeordnet werden, dass sie ein Scharnier bilden.

Das Trägermaterial ist auf der dem Grundkörper im montierten Zustand zugewandten Seite mit einem Kunststoff aufkaschiert, so dass sich eine verbesserte Verbindbarkeit mit dem Material des Grundkörpers realisieren lässt. Der aufkaschierte Kunststoff und der Grundkörper sind vorteilhafterweise eine Materialpaarung, die gut miteinander verschweißbar oder verklebbar ausgebildet sind. Durch den aufkaschierten Kunststoff ist es möglich, dass das Trägermaterial nicht auf einer Verbindung mit dem Grundkörper hin zu optimiert werden braucht, vielmehr kann beispielsweise die Verbindung mit dem Stabilisierungselement optimiert werden, während der aufkaschierte Kunststoff die Verbindung zu dem Grundkörpermaterial herstellt. Durch das Aufbringen eines Kunststoffes werden somit zwei Materialien zwischen das Stabilisierungselement und den Grundkörper geschaltet, so dass sich die unterschiedlichen mechanischen Anforderungen an das Stabilisierungselement und an den Grundkörper leichter realisieren lassen. Der aufkaschierte Kunststoff ist vorteilhafterweise von dem Material des Trägermaterials verschieden und kann als Polyurethan ausgebildet sein. Ebenfalls ist es möglich, dass der aufkaschierte Kunststoff als Dipol-Werkstoff ausgebildet sein.

Die Erfindung sieht vor, dass nur in den über den Umfang der Stabilisierungselemente hinausstehenden Bereichen bzw. in den Bereichen mit verminderten Dicken eine Verklebung oder eine stoffschlüssige Verbindung mit dem Grundkörper ausgeführt ist, so dass die ausreichende Elastizität und Anbindung an den Grundkörper gewahrt ist.

Das Trägermaterial steht über den Umfang des Stabilisierungselementes zumindest abschnittsweise hinaus, so dass Bereiche entstehen, die zur Verklebung oder zum Verschweißen mit dem Grundkörper geeignet sind. Durch das Hinausstehen über die Stabilisierungselemente wird zudem die Kontaktfläche vergrößert, so dass eine stabile Festlegung des Stabilisierungselementes an dem Grundkörper gewährleistet wird.

Das Trägermaterial kann als Textil, insbesondere als ein Velours ausgebildet sein, hier ist insbesondere ein Polyamid-Velours vorteilhaft, da sich in der Veloursoberfläche das Material des Stabilisierungselementes ebenso wie das des aufkaschierten Kunststoffes sehr gut verankert und somit eine gute Anhaftung und Verbindung zwischen dem Stabilisierungselement und dem Trägermaterial ebenso wie dem aufkaschierten Kunststoff und dem Trägermaterial verwirklicht werden kann.

Das Stabilisierungselement kann aus einem Material bestehen, das einen niedrigeren Schmelzpunkt als das Material des Grundkörpers oder des Trägermaterials aufweist. Dadurch ist es möglich, das Stabilisierungselement auf das Trägermaterial oder den Grundkörper aufzuspritzen, ohne dass das Trägermaterial oder der Grundkörper aufgrund des Wärmeeintrages verformt wird oder sich Materialeigenschaften des Trägermaterials oder des Grundkörpers verändern. Das Stabilisierungselement kann beispielsweise aus Polyurethan oder Polyethylen bestehen.

Eine Weiterbildung der Erfindung sieht vor, dass mehrere Stabilisierungselemente gelenkig miteinander verbunden an dem Grundkörper festgelegt sind. Die Festlegung erfolgt über das Trägermaterial. Dazu sind die Stabilisierungselemente so ausgebildet, dass sie eine formschlüssige Verbindung miteinander eingehen, beispielsweise indem ein Stabilisierungselement als eine Öse ausgebildet ist, in die ein Zapfen des zweiten Stabilisierungselementes eingreift. Die Montage der beiden Stabilisierungselemente zu einem Gelenk erfolgt vorteilhafterweise vor dem Befestigen des Trägermaterials an dem Grundkörper, kann jedoch auch nachträglich erfolgen, wenn die beiden Stabilisierungselemente entsprechend an dem Grundkörper positioniert und festgelegt sind. Der Grundkörper ist vorteilhafterweise als Textil, insbesondere als elastisches Textil, beispielsweise als elastisches Gestrick oder elastisches Gewebe ausgebildet. Das Textil kann auf der Innenseite und/oder der Außenseite beschichtet sein, um Eigenschaften der Manschette, zum Beispiel des Liners oder der Bandage, wie gewünscht einzustellen. So können die Innen- und Außenseite luftdicht und/oder haftend beschichtet sein, um eine gute Anlage auf dem Körperteil oder einem Stumpf zu erreichen. Der Grundkörper kann, alternativ dazu kann der Grundkörper auch aus Schaumstoff ausgebildet sein, beispielsweise aus Neopren. Ebenfalls ist die Ausgestaltung der Manschette ganz oder teilweise aus Kunststoff oder Silikon möglich, insbesondere bei einer Ausgestaltung der Manschette als Liner.

Um eine gute Anhaftung des Stabilisierungselementes zu ermöglichen, ist das Trägermaterial vollflächig mit dem Stabilisierungselement verbunden.

Eine Ausführungsform der Erfindung sieht vor, dass die Manschette als Liner ausgebildet ist, wobei das Trägermaterial eine haftende, insbesondere luftdichte Beschichtung aufweist. Diese Beschichtung kann auf der Innenseite und/oder der Außenseite aufgebracht sein. Das Stabilisierungselement kann als distale Kappe oder Teil einer anderen Abstützeinrichtung oder Befestigungseinrichtung zur Festlegung oder Aufnahme eines Prothesenschaftes ausgebildet sein. Der Liner kann einen geschlossenen Querschnitt aufweisen. Alternativ ist die Manschette als Bandage ausgebildet, die zur Anlage an den Körper ausgebildet ist. Die Bandage kann einen geschlossenen Querschnitt aufweisen und über ein Körperglied gezogen werden. Ebenso ist es möglich, dass die Manschette als Bandage mit einem offenen Querschnitt ausgebildet ist und nach dem Anlegen geschlossen oder in der gewünschten Position fixiert wird.

Das Verfahren zum Herstellen einer voranstehend beschriebenen Manschette umfasst die Schritte des Aufspritzens oder Hinterspritzens eines Stabilisierungselementes aus einem Kunststoff auf ein flexibles Trägermaterial, so dass das Trägermaterial zumindest abschnittsweise über dem Umfang des Stabilisierungselementes hinaussteht und das anschließende Verschweißen und/oder Verkleben des Trägermaterials auf einem flexiblen Grundkörperzuschnitt wobei die dem Stabilisierungselement abgewandte Seite des Trägermaterials vor dem Verschweißen und Verkleben mit einem Kunststoff, insbesondere Polyurethan kaschiert wird, um eine verbesserte Verbindung zwischen dem Trägermaterial und dem Grundkörper realisieren zu können und das Trägermaterial mit dem Grundkörperzuschnitt nur in einem über das Stabilisierungselement hinausstehenden Bereich verbunden wird, so dass eine erhöhte Elastizität erreicht und eine unmittelbare Verbindung des Stabilisierungselementes mit dem Grundkörper vermieden wird. Der Grundkörper oder der Grundkörperzuschnitt können sowohl geschlossen als auch offen ausgebildet sein, beispielsweise als zu schließende Lumbalorthese oder als eine geschlossene Kniebandage, die über den Unterschenkel gezogen und angelegt wird. Neben einer beidseitig offenen Ausgestaltung der Manschette, bei der ein im Wesentlichen rohrförmiger Grundkörper vorhanden ist, kann auch eine einseitig geschlossene Manschette vorgesehen sein, die sich beispielsweise über einen Stumpf erstreckt und zwischen dem Körperglied und einer Protheseneinrichtung angeordnet werden kann.

Mehrere Stabilisierungselemente können auf einem Trägermaterialzuschnitt aufgespritzt und anschließend von dem Trägermaterialzuschnitt unter Ausbildung zumindest eines über den Umfang des Stabilisierungselementes hinausstehenden Bereiches des Trägermaterials getrennt werden. Dadurch ist es möglich, die Stabilisierungselemente separat automatisiert herzustellen und bei Bedarf die Stabilisierungselemente aus einem Grundmaterial auszuschneiden und anschließend mit dem Grundkörper zu verbinden.

Der aufkaschierte Kunststoff weist vorteilhafterweise thermoplastische Eigenschaften auf, um auch nach einem thermischen Verbinden eine ausreichende Elastizität bereitzustellen.

Das Trägermaterial kann mit dem Grundkörperzuschnitt mittels einer Hochfrequenzverschweißung verbunden werden, wodurch sich eine erhöhte Variabilität der Nahtbreite durch eine entsprechende Elektrodenführung realisieren lässt. Je nach Belastung kann dann eine schmale oder breite Verbindung des Trägermaterials mit dem Grundkörper erreicht werden.

Nachfolgend werden Ausführungsbeispiele der Erfindung anhand der beigefügten Figuren näher erläutert. Es zeigen:
- Figur 1 -: eine Querschnittsansicht eines Grundkörperzuschnittes mit einem aufgeschweißten Stabilisierungselement;
- Figur 2-: eine Querschnittsdarstellung eines Stabilisierungselementes auf einem Trägermaterial;
- Figur 3 -: eine Querschnittsansicht eines montierten Stabilisierungselementes mit Trägermaterial auf einem Grundkörper;
- Figur 4 -: eine Variante der Figur 3;
- Figur 5 -: eine Kniebandage mit einem aufgebrachten Stabilisierungselement;
- Figur 6 -: eine Variante der Figur 5;
- Figur 7 -: eine Rückenorthese mit aufgebrachten Stabilisierungselementen;
- Figur 8 -: eine Sprunggelenkorthese mit aufgebrachten Stabilisierungselementen;
- Figur 9 -: eine Kniebandage mit mehreren, gelenkig verbundenen Stabilisierungselementen;
- Figur 10 -: eine perspektivische Ansicht einer Knieorthese;
- Figur 11 -: eine Knieorthese gemäß Figur 9 aus anderem Blickwinkel;
- Figur 12 -: Schnittdarstellung der Festlegung von Stabilisierungselementen an einem Grundkörper; sowie
- Figur 13 -: eine Manschette in Gestalt eines Liners in Schnittdarstellung.

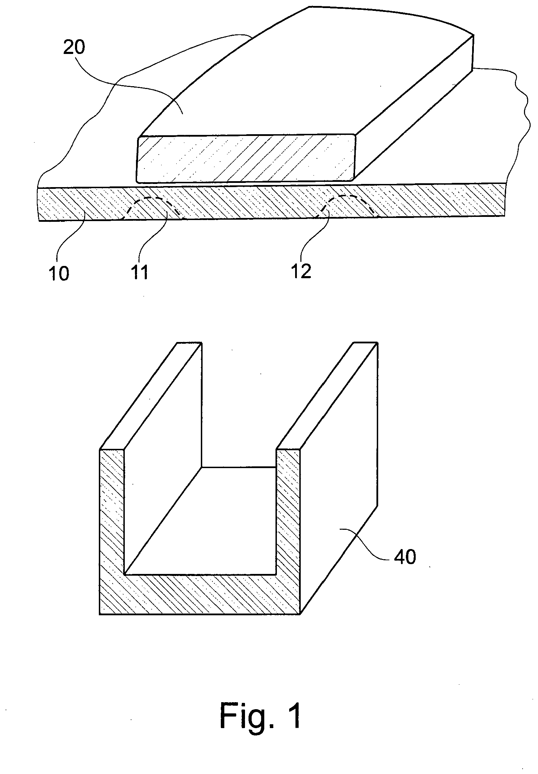

In der Figur 1 ist eine Schnittansicht durch eine herkömmliche Manschette in Gestalt einer Bandage mit einem Grundkörper 10 aus einem Kunststoff oder Textil und einem darauf angebrachten Stabilisierungselement 20 dargestellt. Der Grundkörper 10 und das Stabilisierungselement 20 sind aus einem miteinander verschweißbaren Material hergestellt. Unterhalb des Grundkörpers 10 ist eine Hochfrequenz-Elektrode 40 angeordnet, über die es möglich ist, das Stabilisierungselement 20 auf dem Grundkörper 10 zu befestigen. Befestigungsnähte 11, 12 entlang der Kontaktflächen der Elektrode 40 auf dem Grundkörper 10 sind durch die Strichlinien angedeutet.

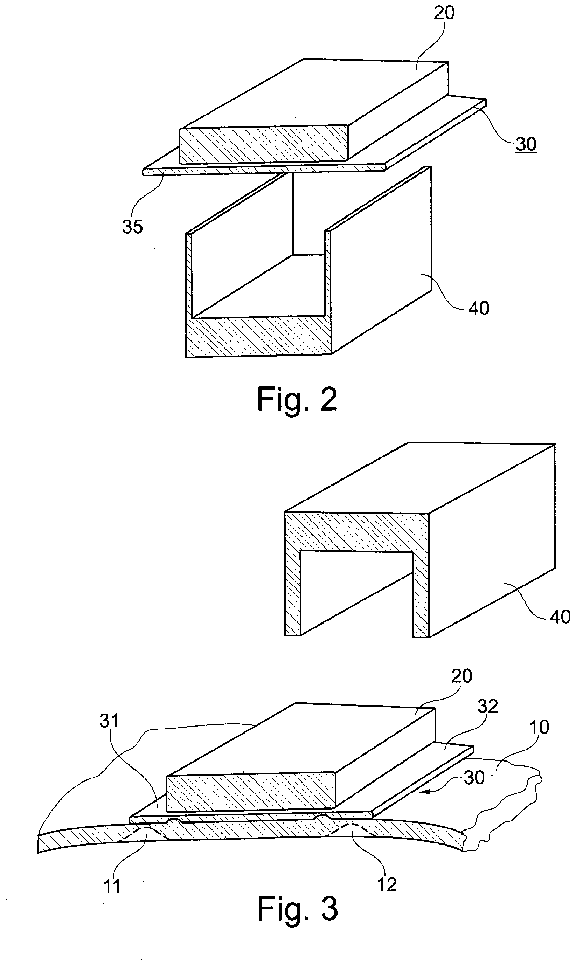

In der Figur 2 ist ein Stabilisierungselement 20 dargestellt, das mit einem Trägermaterial 30 verbunden, insbesondere verschweißt ist. Das Stabilisierungselement 20 kann aus jedem geeigneten Kunststoff bestehen, beispielsweise aus TPU, PVC oder PA. Das Trägermaterial 30 ist im dargestellten Ausführungsbeispiel als eine Kunststofffolie ausgebildet, die aus TPU oder PA hergestellt sein kann. Das Stabilisierungselement 20 und das Trägermaterial 30 sind miteinander verschweißt, beispielsweise durch die Elektrode 40. Alternativ zu einer nur partiellen Verschweißung kann das Stabilisierungselement 20 mit dem Trägermaterial 30 vollflächig verschweißt werden. Auf der dem Stabilisierungselement 20 abgewandten Oberfläche des Trägermaterials 30 kann eine Kunststoffschicht 35 aufkaschiert sein, die von dem Material des Trägermaterials 30 verschieden ausgebildet sein kann. Ebenfalls ist es möglich, dass das Stabilisierungselement 20 auf dem Trägermaterial 30 verklebt ist, wobei auch hier eine vollflächige oder nur partielle Verklebung vorgesehen sein kann.

Alternativ zu einer Ausgestaltung des Trägermaterials 30 als eine Folie kann dies auch als ein Textil ausgebildet sein, auf dem das Stabilisierungselement 20 befestigt ist. Die Befestigung des Stabilisierungselementes auf einem textilen Trägermaterial 30 kann ebenfalls über Verschweißen, Verkleben oder Anspritzen oder Hinterspritzen erfolgen.

Alternativ zu einer nachträglichen Anbringung des Trägermaterials 30 an dem Stabilisierungselement 20 kann die Herstellung eines Stabilisierungselementes 20 mit einem daran angeordneten Trägermaterial durch Zwei-Komponenten-Spritzgießen erfolgen.

In der Figur 3 ist gezeigt, wie das Stabilisierungselement 20 zusammen mit dem Trägermaterial 30 auf einem Grundkörper 10 oder einem Grundkörperzuschnitt aufgebracht ist. Seitlich neben dem Stabilisierungselement 20 sind zwei Bereiche 31, 32 des Trägermaterials 30 vorgesehen, die über den Umfang des Stabilisierungselementes 20 hinausstehen. Entlang dieser Bereiche 31, 32 erfolgt eine Verschweißung des Trägermaterials 30 mit dem Grundkörper 10, der als Textil, beispielsweise als elastisches Gestrick oder Gewirk, oder aus Schaumstoff, beispielsweise aus Neopren, ausgebildet sein kann. Die Schweißnähte 11, 12 sind gestrichelt in dem Grundkörper 10 angedeutet.

Durch die Ausstattung des Stabilisierungselementes 20, das aus Kunststoff ausgebildet ist oder zumindest eine Kunststoffschicht an der Unterseite zum Verbinden mit dem Trägermaterial aufweist, mit dem Trägermaterial 30 ist es möglich, ein an das Material des Grundkörpers 10 angepasstes Kopplungselement bereitzustellen, so dass eine direkte stoffschlüssige oder adhäsive Verbindung des in der Regel steifen Stabilisierungselementes 20 mit dem in der Regel flexiblen Grundkörper 10 vermieden wird. Durch die indirekte Kopplung des Stabilisierungselementes 20 mit dem Grundkörper 10 wird die Gefahr der Rissbildung verringert, ebenso können optimierte Materialeigenschaften für den Grundkörper 10 und das Stabilisierungselement 20 realisiert werden, ohne Rücksicht auf eine grundsätzliche Verbindbarkeit der beiden Komponenten miteinander nehmen zu müssen. Es kann beispielsweise das Stabilisierungselement 20 eine hohe Biegefestigkeit aufweisen, während der Grundkörper 10 hinsichtlich der Tragbarkeit, Hautverträglichkeit und Elastizität optimiert ausgebildet werden kann.

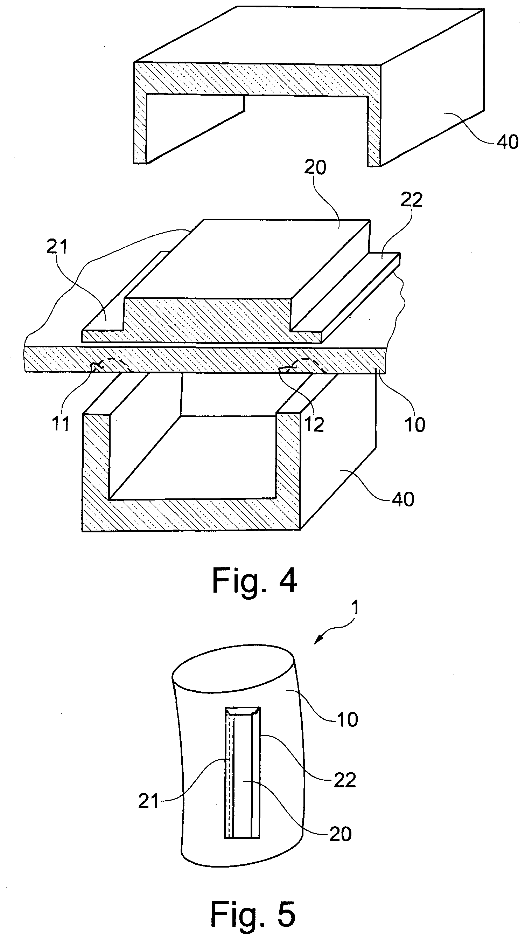

Eine Variante, die nicht Gegenstand der Erfindung ist, ist in der Figur 4 dargestellt, bei der statt einer zweiteiligen Ausführung des Stabilisierungselementes 20 mit dem Trägermaterial 30 ein einteiliges Stabilisierungselement 20 vorgesehen ist, das in Längserstreckung gesehen seitliche Bereiche 21, 22 aufweist, die einen geringeren Querschnitt als der dazwischenliegende Bereich des Stabilisierungselementes 20 aufweisen. Die seitlichen Bereiche 21, 22 ermöglichen eine vergrößerte Elastizität und Beweglichkeit, so dass insbesondere bei einer Hochfrequenzverschweißung über die Elektroden 40 entlang der seitlichen Bereiche 21, 22 und einer Anbindung entlang der Schweißnähte 11, 12 nur eine geringfügige Versteifung des Grundkörpers 10 in dem Verbindungsbereich erfolgt. Dadurch wird die Gefahr der Ablösung des Stabilisierungselementes 20 von dem Grundkörper 10 aufgrund von voneinander abweichenden Biegsamkeiten reduziert.

In der Figur 5 ist schematisch eine geschlossene Bandage 1 mit einem im Wesentlichen zylindrischen, gegebenenfalls vorflektierten Grundkörper 10 dargestellt, auf dessen Außenseite ein Stabilisierungselement 20 angebracht ist. Das Stabilisierungselement 20 entspricht dabei dem Stabilisierungselement 20 der Figur 4 und ist einteilig aufgebaut und entlang der dünneren Bereiche 21, 22 an dem Grundkörper 10 befestigt. Die Befestigung kann über Kleben oder Verschweißen erfolgen.

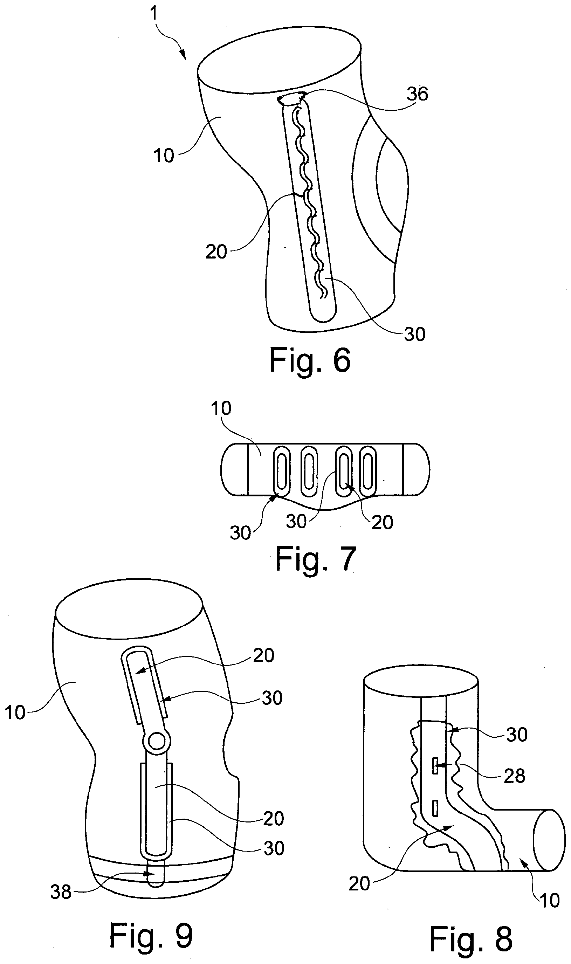

Eine Variante der Erfindung ist in der Figur 6 in Gestalt einer Kniebandage dargestellt. Seitlich an der Außenseite des Grundkörpers 10, der geschlossen ausgebildet ist, ist ein Stabilisierungselement 20 in Gestalt einer Kunststofffeder angeordnet. Die Kunststofffeder ist auf einem Trägermaterial 30, das als Textil, insbesondere als ein Velours ausgebildet sein kann, aufgebracht. Das Aufbringen auf dem Trägermaterial 30 kann beispielsweise über Anspritzen, Hinterspritzen, Hochfrequenzverschweißen oder Ankleben erfolgen. Über das Trägermaterial 30 wird die Kunststofffeder dann an dem Grundkörper 10 befestigt, beispielsweise über ein Verkleben oder ein Hochfrequenzverschweißen.

An dem Trägermaterial können Zusatzkomponenten 36 vorgesehen sein, beispielsweise ein Klettverschluss oder eine Anziehschlaufe, die bei dem Herstellvorgang des Trägermaterials 30, gegebenenfalls in Verbindung mit der Herstellung der Verbindung zu dem Stabilisierungselement 20 angebracht oder ausgebildet werden können. Das Stabilisierungselement 20 kann zusammen mit dem Trägermaterial 30 automatisiert vorgefertigt werden, so dass eine Reihe von Stabilisierungselementen 20 beabstandet zueinander auf einem Trägermaterialzuschnitt angebracht werden können. Aus diesem Zuschnitt mit den aufgebrachten Stabilisierungselementen können dann je nach Bedarf ein geeignetes Stabilisierungselement oder mehrere Stabilisierungselemente zusammen mit einem Trägermaterialüberstand herausgeschnitten werden. Eine Variante der Erfindung ist in der Figur 7 dargestellt, bei der die Manschette 1 als eine Rückenorthese ausgebildet ist, die einen offenen Grundkörperzuschnitt aufweist, an dessen Enden Verbindungselemente, beispielsweise in Gestalt eines Klettverschlusses, ausgebildet sind, über die die Rückenorthese geschlossen werden kann. An der Außenseite sind über das Trägermaterial 30 Stabilisierungselemente 20 beabstandet zueinander an den Grundkörper 10 festgelegt.

Eine weitere Variante der Erfindung ist in der Figur 8 gezeigt, in der eine Sprunggelenksorthese dargestellt ist. An der Außenseite medial und lateral ist jeweils ein Stabilisierungselement 20 angeordnet, das an einem Trägermaterial 30 befestigt ist. Der Grundkörper kann, wie auch bei den anderen als Bandagen ausgebildeten Manschetten, aus einem Schaumstoff, beispielsweise Neopren, oder einem elastischen Gestrick oder Gewirk bestehen. Das Trägermaterial 30 kann aus einem Velours, einem anderen Textil oder ebenfalls einem geeigneten Kunststoff, beispielsweise einer Kunststofffolie oder einem Schaumstoff, bestehen. An der Außenseite des Stabilisierungselementes 20 kann ein Flauschbereich 28 oder ein Klettbereich befestigt sein, um eine zusätzliche Stabilisierung über Gurte oder dergleichen realisieren zu können.

Eine weitere Variante der Erfindung ist in der Figur 9 dargestellt, bei der die Manschette 1 als Knieorthese ausgebildet ist. Auch hier sind an der Außenseite medial und lateral Stabilisierungselemente 20 über ein Trägermaterial 30 an dem geschlossenen, schlauchförmigen und vorflektierten Grundkörper 10 befestigt. Das Trägermaterial 30 ist partiell mit dem Grundkörper 10 verbunden. An der dem Grundkörper 10 abgewandten Seite des Trägermaterials 30 sind Stabilisierungselemente 20 angeordnet, die so geformt sind, dass sie eine gelenkige Verbindung ausbilden können. Das obere Stabilisierungselement 20 weist eine ringförmige Ausnehmung auf, in die ein entsprechend ausgebildeter Vorsprung des unteren Stabilisierungselementes 20 eingreift, so dass ein Scharniergelenk entsteht, das vorzugsweise im Bereich der natürlichen Knieachse des Orthesenträgers positioniert ist. Das Trägermaterial 30 kann einen Verklettungspunkt 38 an der Außenseite aufweisen, beispielsweise aus Velours, an dem ein Hakenbereich eines Gurtes befestigt werden kann.

Die dem Grundkörper 10 zugewandte Seite des Trägermaterials 30 kann mit einer TPU-Folie kaschiert sein, die über einen indirekten Wärmeeintrag, beispielsweise über hochfrequente oder niedrigfrequente Erregung erzeugt werden kann, aufgeschmolzen und mit dem Material des Grundkörpers 10 verbunden werden.

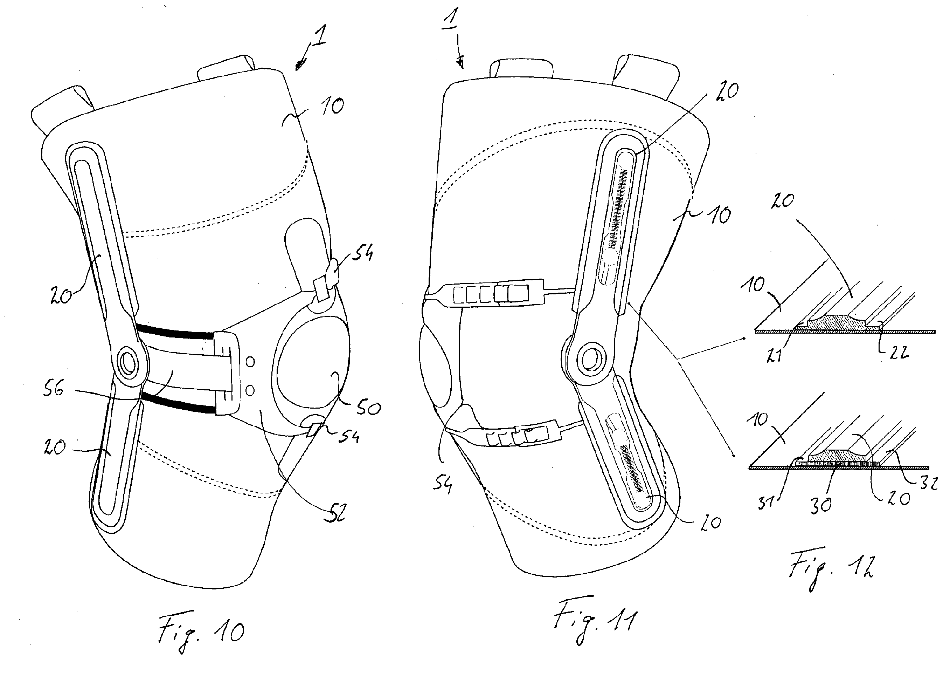

Figur 10 zeigt eine weitere Variante einer Manschette 1 in Gestalt einer Knieorthese mit einem flexiblen, elastischen Grundkörper 10 aus einem Textil, gegebenenfalls einem beschichteten Textil, mit zwei lateral angeordneten Stabilisierungselementen 20, die gelenkig miteinander verbunden sind. Die Gelenkverbindung liegt vorteilhafterweise im Bereich der natürlichen Gelenkachse und wird vorteilhafterweise dort positioniert. In dem Grundmaterial 10 ist frontal eine Ausnehmung 50 eingearbeitet, die im Bereich der Patella ausgebildet ist, so dass durch das elastische Material des Grundkörpers 10 kein Druck auf die Patella ausgeübt wird. Ein Stützelement 52, das seitlichen Druck auf die Patella ausübt, um die Position der Patella zu korrigieren. Gehalten wird das Stützelement 52 durch einen ersten Gurt 56, der im Bereich der Gelenkverbindung der Stabilisierungselemente 20 festgelegt ist.

Die Figur 11 zeigt die Bandage 1 aus medialer Sicht, zwei zweite Gurte 54 sind an dem Stützelement 52 befestigt, um die Position in medialer Richtung zu fixieren.

In den Darstellungen der Figur 12 sind Detailansichten von Querschnitten durch das Stabilisierungselement 20 und den Grundkörper 10 dargestellt. In der oberen Darstellung der Figur 12, die nicht Gegenstand der Erfindung ist, ist das Stabilisierungselement 20 in Gestalt einer Kunststoffschiene mit seitlich der Längserstreckung des Stabilisierungselementes 20 ausgebildeten Bereichen 21, 22, entlang der das Stabilisierungselement 20 an dem Grundkörper 10 befestigt ist. Die Befestigung erfolgt zum Beispiel durch Hochfrequenzverschweißen oder durch Verkleben.

In der unteren Darstellung der Figur 12 ist eine erfindungsgemäße Variante der Befestigung gezeigt, bei der zwischen dem Stabilisierungselement 20 und dem Grundkörper 10 ein Trägermaterial 30 angeordnet ist, das seitlich neben dem Stabilisierungselement 20 Bereiche 31, 32 aufweist, in denen eine Verschweißung des Trägermaterials mit dem Grundkörper 10 erfolgt.

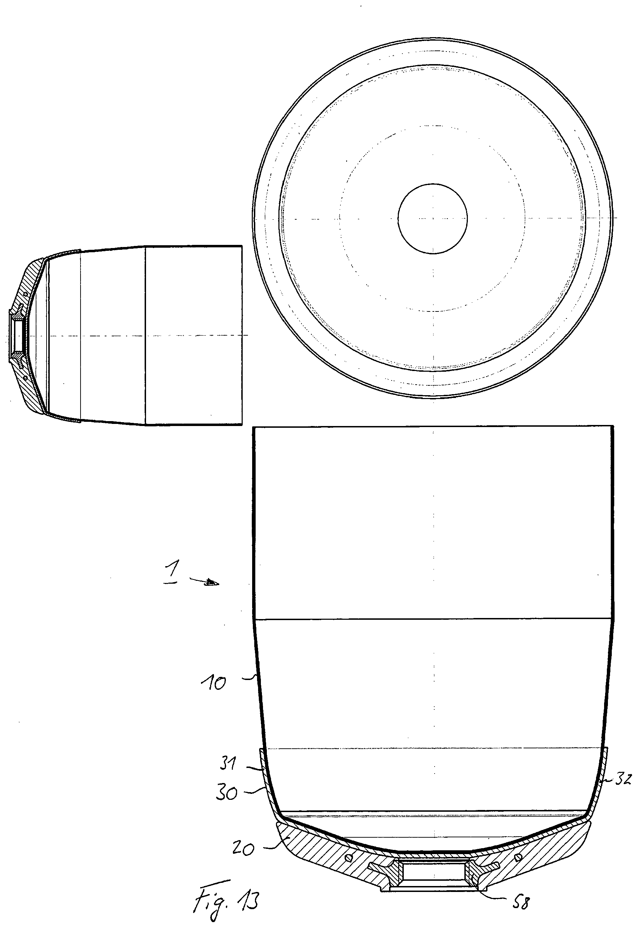

Eine Variante der Erfindung ist in der Figur 13 dargestellt, in der eine Manschette 1 in Gestalt eines Liners in Querschnittsansicht gezeigt ist. Der Grundkörper 10 besteht aus einem im Wesentlichen rohrförmigen Kunststoffelement, das einen geschlossenen Querschnitt aufweist. Einer proximalen Öffnung gegenüberliegend ist ein geschlossener distaler Abschnitt des Grundkörpers 10 vorgesehen. Der Grundkörper 10 ist aus einem luftdichten Kunststoff oder Silikonmaterial ausgebildet und nimmt einen Stumpf einer Gliedmaße auf. An dem distalen Ende des Grundkörpers 10 ist eine Linerkappe als Stabilisierungselement 20 angeordnet. Innerhalb dieses Stabilisierungselementes 20 ist ein Einsatz 58 angeordnet, in den Befestigungseinrichtungen zum Festlegen beispielsweise an einem Prothesenschaft eingeschraubt werden können. Weiterhin sieht das Stabilisierungselement 20 ein flexibles Trägermaterial 30 vor, das an der Linerkappe befestigt ist. Das Trägermaterial 30 steht seitlich über das Stabilisierungselement 20 hinaus und erstreckt sich in proximaler Richtung auf die Öffnung des Grundkörpers 10 hin, so dass das distale Ende des Grundkörpers 10 vollständig von dem Trägermaterial 30 eingefasst ist. Die seitlich sich zum proximalen Ende erstreckenden Bereiche 31, 32 sind mit dem Grundkörper 10 stoffschlüssig verbunden, beispielsweise angeschweißt oder verklebt.

## Patentansprüche

1. Orthopädietechnische Manschette zur Anlage an einen Körper mit einem flexiblen Grundkörper (10) mit zumindest einem daran angeordneten Stabilisierungselement (20) aus Kunststoff, das auf einem flexiblen Trägermaterial (30) aufgebracht ist, das auf der dem Grundkörper (10) zugewandten Seite mit einem mit dem Grundkörper (10) verschweißbaren oder verklebbaren Kunststoff (35) kaschiert ist, **dadurch gekennzeichnet, dass** das Trägermaterial (30) über den Umfang des Stabilisierungselementes (20) zumindest abschnittsweise hinaussteht und nur in über den Umfang des Stabilisierungselementes (20) hinausstehenden Bereichen (31, 32) des Trägermaterials (30) eine Verklebung oder andere stoffschlüssige Verbindung mit dem Grundkörper (10) ausgeführt ist.

2. Orthopädietechnische Manschette nach Anspruch 1, **dadurch gekennzeichnet, dass** der aufkaschierte Kunststoff (35) von dem Material des Trägermaterials (30) verschieden und insbesondere ein Polyurethan ist.

3. Orthopädietechnische Manschette nach einem der voranstehenden Ansprüche, **dadurch gekennzeichnet, dass** das Trägermaterial (30) als Textil, insbesondere als ein Velours, insbesondere als ein Polyamid-Velours ausgebildet ist.

4. Orthopädietechnische Manschette nach einem der voranstehenden Ansprüche, **dadurch gekennzeichnet, dass** das Stabilisierungselement (20) aus einem Material besteht, das einen niedrigeren Schmelzpunkt als der Grundkörper (10) oder das Trägermaterial (30) aufweist.

5. Orthopädietechnische Manschette nach einem der voranstehenden Ansprüche, **dadurch gekennzeichnet, dass** das Stabilisierungselement (20) aus Polyurethan oder Polyethylen besteht.

6. Orthopädietechnische Manschette nach einem der voranstehenden Ansprüche, **dadurch gekennzeichnet, dass** mehrere Stabilisierungselemente (20) gelenkig miteinander verbunden an dem Grundkörper (10) festgelegt sind.

7. Orthopädietechnische Manschette nach einem der voranstehenden Ansprüche, **dadurch gekennzeichnet, dass** der Grundkörper (10) aus Textil, insbesondere als elastisches Textil, insbesondere als elastisches Gestrick oder elastisches Gewebe, aus Kunststoff, Silikon oder aus Schaumstoff ausgebildet ist.

8. Orthopädietechnische Manschette nach einem der voranstehenden Ansprüche, **dadurch gekennzeichnet, dass** das Trägermaterial (30) vollflächig mit dem Stabilisierungselement (20) verbunden ist.

9. Orthopädietechnische Manschette nach einem der voranstehenden Ansprüche, **dadurch gekennzeichnet, dass** die Manschette als Liner ausgebildet ist, das Trägermaterial eine haftende, insbesondere luftdichte Beschichtung aufweist und das Stabilisierungselement (20) als distale Kappe ausgebildet ist oder dass der Grundkörper (10) als eine Bandage ausgebildet ist.

10. Orthopädietechnische Manschette nach einem der voranstehenden Ansprüche, **dadurch gekennzeichnet, dass** das Stabilisierungselement (20) auf dem flexiblen Trägermaterial (30) aufgespritzt oder hinterspritzt ist.

11. Verfahren zum Herstellen einer orthopädietechnischen Manschette nach einem der voranstehenden Ansprüche, umfassend folgende Schritte:
• Aufspritzen oder Hinterspritzen eines Stabilisierungselementes (20) aus einem Kunststoff auf ein flexibles Trägermaterial (30), so dass das Trägermaterial (30) zumindest abschnittsweise über den Umfang des Stabilisierungselementes (20) hinaussteht;
• Verschweißen und/oder Verkleben des Trägermaterials (30) auf einem flexiblen Grundkörperzuschnitt,
• wobei die dem Stabilisierungselement (20) abgewandte Seite des Trägermaterials (30) vor dem Verschweißen oder Verkleben mit einem Kunststoff (35), insbesondere Polyurethan, kaschiert und das Trägermaterial (30) mit dem Grundkörper (10) nur an einem über das Stabilisierungselement (20) hinausstehenden Bereich (31, 32) verbunden wird.

12. Verfahren nach Anspruch 11, **dadurch gekennzeichnet, dass** mehrere Stabilisierungselemente (20) auf einem Trägermaterialzuschnitt aufgespritzt und anschließend von dem Trägermaterialzuschnitt unter Ausbildung zumindest eines über den Umfang des Stabilisierungselementes (20) hinausstehenden Bereiches (31, 32) des Trägermaterials (30) getrennt werden.

13. Verfahren nach einem der Ansprüche 11 oder 12, **dadurch gekennzeichnet, dass** das Trägermaterial (30) mit dem Grundkörperzuschnitt mittels Hochfrequenzverschweißung verbunden wird.

## Claims

1. Orthopedic sleeve for placing on a body, comprising a flexible main body (10) having at least one plastics stabilizing element (20) which is arranged thereon, the stabilizing element is applied to a flexible support material (30) that is laminated on the side facing the main body (10) with a plastics material (35) that can be welded or bonded to the main body (10), **characterized in that** the support material (30) projects beyond the periphery of the stabilizing element (20) at least in portions, and a bond or another kind of integral connection with the main body (10) is provided only in regions (31, 32) of the support material (30) which project beyond the periphery of the stabilizing element (20).

2. Orthopedic sleeve according to claim 1, **characterized in that** the laminated-on plastics material (35) is different from the material of the support material (30), and is in particular polyurethane.

3. Orthopedic sleeve according to either of the preceding claims, **characterized in that** the support material (30) is a textile, in particular a velour, in particular a polyamide velour.

4. Orthopedic sleeve according to any of the preceding claims, **characterized in that** the stabilizing element (20) consists of a material that has a lower melting point than the main body (10) or the support material (30).

5. Orthopedic sleeve according to any of the preceding claims, **characterized in that** the stabilizing element (20) consists of polyurethane or polyethylene.

6. Orthopedic sleeve according to any of the preceding claims, **characterized in that** a plurality of stabilizing elements (20) are secured to the main body (10) in a hingedly interconnected manner.

7. Orthopedic sleeve according to any of the preceding claims, **characterized in that** the main body (10) is a textile, in particular a resilient textile, in particular a resilient knitted fabric or a resilient woven fabric or is made of plastics material, silicone or foam.

8. Orthopedic sleeve according to any of the preceding claims, **characterized in that** the support material (30) is connected to the stabilizing element (20) over the full surface thereof.

9. Orthopedic sleeve according to any of the preceding claims, **characterized in that** the sleeve is a liner, the support material has an adherent, in particular air-tight coating, and the stabilizing element (20) is a distal cap, or **in that** the main body (10) is a bandage.

10. Orthopedic sleeve according to any of the preceding claims, **characterized in that** the stabilizing element (20) injection molded onto or from behind the flexible support material (30).

11. Method for producing an orthopedic sleeve according to any of the preceding claims, comprising the following steps:
• injection molding a plastic stabilizing element (20) onto or from behind a flexible support material (30) such that the support material (30) projects beyond the periphery of the stabilizing element (20) at least in portions;
• welding and/or bonding the support material (30) to a flexible main body blank,
• wherein the side of the support material (30) that faces away from the stabilizing element (20) is laminated with a plastics material (35), in particular polyurethane, before the welding or bonding, and the support material (30) is connected to the main body (10) only at a region (31, 32) which projects beyond the stabilizing element (20).

12. Method according to claim 11, **characterized in that** a plurality of stabilizing elements (20) are molded onto a support material blank and are subsequently separated from the support material blank so as to form at least one region (31, 32) of the support material (30) that projects beyond the periphery of the stabilizing element (20).

13. Method according to either claim 11 or claim 12, **characterized in that** the support material (30) is connected to the main body blank by means of high-frequency welding.

## Revendications

1. Manchette orthopédique destinée à être appliquée sur un corps, comportant un corps de base flexible (10) pourvu d'au moins un élément de stabilisation (20) agencé sur ce dernier et constitué en matière plastique et déposé sur un matériau porteur flexible (30) qui, sur le côté tourné vers le corps de base (10), est doublé d'une matière plastique (35) susceptible d'être soudée ou collée sur le corps de base (10),
**caractérisée en ce que**
le matériau porteur (30) dépasse au moins localement au-delà de la périphérie de l'élément de stabilisation (20), et un collage ou une autre liaison par coopération de matière avec le corps de base (10) est réalisée uniquement dans des zones (31, 32) du matériau porteur (30) qui dépassent au-delà de la périphérie de l'élément de stabilisation (20).

2. Manchette orthopédique selon la revendication 1,
**caractérisée en ce que**
la matière plastique (35) doublée diffère du matériau porteur (30) et est en particulier un polyuréthane.

3. Manchette orthopédique selon l'une des revendications précédentes,
**caractérisée en ce que**
le matériau porteur (30) est réalisé sous forme de textile, en particulier de velours, en particulier d'un velours en polyamide.

4. Manchette orthopédique selon l'une des revendications précédentes,
**caractérisée en ce que**
l'élément de stabilisation (20) est constitué d'un matériau qui présente un point de fusion inférieur à celui du corps de base (10) ou du matériau porteur (30).

5. Manchette orthopédique selon l'une des revendications précédentes,
**caractérisée en ce que**
l'élément de stabilisation (20) est constitué en polyuréthane ou en polyéthylène.

6. Manchette orthopédique selon l'une des revendications précédentes,
**caractérisée en ce que**
plusieurs éléments de stabilisation (20) sont immobilisés sur le corps de base (10) en étant reliés en articulation les uns aux autres.

7. Manchette orthopédique selon l'une des revendications précédentes,
**caractérisée en ce que**
le corps de base (10) est réalisé en textile, en particulier sous la forme d'un textile élastique, en particulier sous la forme d'un tricot élastique ou d'un tissu élastique, en matière plastique, en silicone ou en mousse.

8. Manchette orthopédique selon l'une des revendications précédentes,
**caractérisée en ce que**
le matériau porteur (30) est relié par toute la surface à l'élément de stabilisation (20).

9. Manchette orthopédique selon l'une des revendications précédentes,
**caractérisée en ce que**
la manchette est réalisée sous forme de manchon, le matériau porteur comprend un revêtement adhésif, en particulier étanche à l'air, et l'élément de stabilisation (20) est réalisé sous forme de capuchon distal, ou **en ce que**
le corps de base (10) est réalisé sous forme de bandage.

10. Manchette orthopédique selon l'une des revendications précédentes,
**caractérisée en ce que**
l'élément de stabilisation (20) est injecté sur le devant ou sur l'arrière du matériau porteur flexible (20).

11. Procédé pour réaliser une manchette orthopédique selon l'une des revendications précédentes, comprenant les étapes suivantes consistant à :
- injecter un élément de stabilisation (20) en une matière plastique sur le devant ou l'arrière d'un matériau porteur flexible (30), de sorte que le matériau porteur (30) dépasse au moins localement au-delà de la périphérie de l'élément de stabilisation (20) ;
- souder et/ou coller le matériau porteur (30) sur un patron de corps de base flexible,
- doubler la face du matériau porteur (30) détournée de l'élément de stabilisation (20) avec une matière plastique (35), en particulier avec du polyuréthane, avant de la souder ou coller, et relier le matériau porteur (30) au corps de base (10) uniquement sur une zone (31, 32) qui dépasse au-delà de l'élément de stabilisation (20).

12. Procédé selon la revendication 11,
**caractérisé en ce que**
plusieurs éléments de stabilisation (20) sont injectés sur le patron de matériau porteur et sont ensuite séparés du patron de matériau porteur, en réalisant au moins une zone (31, 32) du matériau porteur (30), laquelle dépasse au-delà de la périphérie de l'élément de stabilisation (20).

13. Procédé selon l'une des revendications 11 ou 12,
**caractérisé en ce que**
le matériau porteur (30) est relié au patron de corps de base par soudage à haute fréquence.
